# EUROPÄISCHE PATENTANMELDUNG

(11) **EP 0 601 427 A2**
(43) Veröffentlichungstag der Anmeldung: **15.06.1994**
(21) Anmeldenummer: 93119171.2
(22) Anmeldetag: 29.11.1993
(51) Int. Cl.: A61B 1/00

(54) **Endoskopisches Instrument**

(30) Priorität: 02.12.1992 DE 4241643
(71) Anmelder: Richard Wolf GmbH, D-75438 Knittlingen (DE)
(72) Erfinder: Herrmann, Uwe, Dr. med. Dr. phil., D-40470 Düsseldorf (DE); Boebel, Manfred, D-75443 Oetisheim (DE)
(74) Vertreter: Vollmann, Heiko, Dipl.-Ing.

(57) **Zusammenfassung**

Das endoskopische Instrument (1) weist einen rohrförmigen Schaft (4) zum Einführen einer Betrachtungsoptik (9) und eines Behandlungsinstrumentes (15) auf, der an seinem distalen Endteil einen rinnenförmig auslaufenden Teil (17) aufweist. In diesem rinnenförmigen Endteil (17) ist eine Ausnehmung zur Bildung eines zusätzlichen Ausblickfensters für die Betrachtungsoptik (9) vorgesehen. Damit wird einerseits ein vollständiger Schutz zu dieser Seite des Instrumentes (1) gewährleistet, andererseits aber eine optische Kontrolle in diesem Bereich möglich, was insbesondere bei endoskopischen Eingriffen in der weiblichen Brust (3) zur Kontrolle oder Eröffnung eines Implantates (2) geeignet ist.

## Beschreibung

Die Erfindung betrifft ein endoskopisches Instrument mit den im Oberbegriff des Anspruchs 1 angegebenen Merkmalen.

Die vorliegende Erfindung geht aus von einem Pan-Hysteroskop-System, wie es beispielhaft von der Richard Wolf GmbH in Knittlingen angeboten und vertrieben wird. Bei diesem Instrument liegen nebeneinander angeordnet innerhalb des Schaftes eine Betrachtungsoptik sowie ein Behandlungsinstrument. Da das distale Schaftende in einen rinnenförmigen Teil ausläuft, liegen sowohl Optik als auch Instrument zu einer Seite geschützt während sie zur anderen, also zu der offenen Seite des rinnenförmigen Teils derart offen liegen, daß beispielsweise das Instrument in diese Richtung abgebogen oder auch in seiner Axialrichtung verschoben werden kann. Als Instrument kann beispielsweise eine flexible Zange, eine Laserfaser oder dergleichen eingesetzt werden. Ein solches Instrument ist insbesondere zur endoskopischen Untersuchung und Behandlung der Gebährmutter vorgesehen.

Insbesondere zur Brustrekonstruktion werden seit langer Zeit Silikoneinlagen verwendet. Bei solchen Silikoneinlagen kommt es gelegentlich zur Verkapselung des Implantates. Auch sind Beschädigungen des Implantates nicht auszuschließen. In solchen Fällen war es bisher üblich, auf chirurgischem Wege das Implantat zu überprüfen, die Verkapselung aufzulösen oder ggf. das Implantat auszutauschen. Hiervon ausgehend liegt der vorliegenden Erfindung die Aufgabe zugrunde, ein endoskopisches Instrument so auszubilden, daß sowohl die visuelle Prüfung des Implantates als auch die Lösung einer Verkapselung sowie ggf. weitere Eingriffe insbesondere im Bereich der weiblichen Brust mit genügender Sicherheit endoskopisch und damit minimal invasiv durchgeführt werden können.

Gemäß der Erfindung wird dies ausgehend von dem einleitend genannten Stand der Technik (Hysteroskop) dadurch erreicht, daß in dem distalen rinnenförmig auslaufenden Endteil des Schaftes eine Ausnehmung zur Bildung eines zusätzlichen Ausblickfensters für die Betrachtungsoptik vorgesehen ist.

Das erfindungsgemäße Instrument ermöglicht endoskopische Eingriffe im Bereich der weiblichen Brust, insbesondere im Zusammenhang mit der Inspektion und Lösung einer Verkapselung des Implantates. Natürlich können mit einem solchen Instrument auch andere Eingriffe, wie beispielsweise Gewebeentnahmen oder zu anderen diagnostischen Zwecken erfolgen. Der Operateur führt das erfindungsgemässe Instrument im Bereich der Brustwarze ein und kann dann ohne offene Operation zunächst einmal das Implantat inspizieren und ggf. auch eine Verkapselung eröffnen. Bei allen derartigen endoskopischen Eingriffen ist der Schutz des Implantates besonders zu beachten. Hierfür ist u.a. der rinnenförmige Endteil des Schaftes vorgesehen, der sowohl die Betrachtungsoptik als auch das Behandlungsinstrument, beispielsweise eine Laserfaser, zu einer Seite hin abschirmt. Denn es ist in jedem Falle ein Kontakt zwischen der Laserfaser einerseits und dem Implantat andererseits zu vermeiden. Um dies sicher zu vermeiden, genügt aber die schnabelförmige Ausbildung des distalen Schaftendes allein nicht, denn es ist insbesondere bei der Eröffnung der Verkapselung erforderlich, stets das Implantat im Blick zu haben. Um aber einerseits den erforderlichen Schutz zum Implantat zu gewähren andererseits aber gleichzeitig eine visuelle Kontrolle des Implantates, zumindest eines Teiles davon, durchführen zu können, ist das zusätzliche Ausblickfenster in dem rinnenförmig auslaufenden Endteil des Schaftendes vorgesehen. Der Operateur kann also während des Eröffnens der Verkapselung stets das Implantat beobachten. Die rinnenförmige Ausbildung des Schaftendes gewährleistet bei bestimmungsgemäßer Handhabung zudem, daß das Behandlungsinstrument stets nur in seiner axialen Richtung bzw. in der vom Implantat abgewandten Richtung, also zur offenen Seite des Rinnenprofils hin bewegt und eingesetzt werden kann und daß andererseits das Instrument selbst auf dem Implantat entlanggleitet.

Gemäß der Erfindung wird bevorzugt eine Laserfaser als Behandlungsinstrument eingesetzt, es kann jedoch auch eine HF-Sonde oder ein mechanisches Instrument, beispielsweise eine Zange oder ein Kombinationsinstrument eingesetzt werden. Insbesondere für die vorerwähnte Eröffnung der Kapsel hat sich der Einsatz einer Laserfaser bewährt, die beispielsweise mittels eines Neodym-YAG-Laser gespeist wird. Es versteht sich, daß mit dem erfindungsgemäßen Instrument auch Gewebeentnahmen oder andere Eingriffe vorgenommen werden können.

Bevorzugt weist das Instrument einen im wesentlichen ovalen Querschnitt auf, wobei der rinnenförmige Endteil bezogen auf den übrigen Schaftquerschnitt so ausgebildet und angeordnet ist, daß er eine lange Halbachse des Querschnittovals einschließt. Die ovale Querschnittsform als solche bietet zunächst einmal im Vergleich zu einem runden Querschnitt gleichen Durchmessers den Vorteil eines geringeren Umfangs und damit einer geringeren Belastung der Eingriffsöffnung. In diesem Querschnittsoval können dann Betrachtungsoptik und Behandlungsinstrument so nebeneinander liegend angeordnet werden, daß die Betrachtungsoptik an dem Schaftteil unmittelbar anliegend ist, der zu dem rinnenförmigen Endteil ausläuft, so daß einerseits ein freier Blick durch das zusätzliche Ausblickfenster in diesem Endteil und andererseits ein freier Blick auf das Behandlungsinstrument und die davon behandelte Gewebestelle gewährleistet ist.

Es versteht sich, daß insbesondere das distale Instrumentenende soweit wie möglich gerundet ausgebildet ist, um ein möglichst reibungsloses und verletzungsfreies Gleiten innerhalb des Körpers zu ermöglichen. Bevorzugt ist jedoch der rinnenförmige Endteil des Schaftes zusätzlich mit einer Abschrägung an seiner distalen Stirnseite versehen, um auf diese Weise ein leichteres Vorschieben in Axialrichtung im Körperinneren zu ermöglichen, was durch die zugeführte Spülflüssigkeit noch unterstützt wird.

Insbesondere bei der vorerwähnten Eröffnung einer Implantatverkapselung ist es von Vorteil, wenn sich der rinnenförmige Endteil des Schaftes nicht wie bei Hysteroskopen üblich nur um einen Umfangswinkel von etwa 180°, sondern darüber hinaus bis etwa 200° oder mehr erstreckt, da dann die distalen Enden von Betrachtungsoptik und Behandlungsinstrument noch besser geschützt liegen und somit eine Verletzung des Implantates weitestgehend ausgeschlossen werden kann. Dabei hat es sich besonders bewährt, wenn das Rinnenprofil des distalen Schaftendteils nicht symmetrisch ausgebildet ist, sondern eine Wandung zu einer Seite der langen Achse des Querschnittovals verlängert ist. Bevorzugt liegt dann die Ausnehmung zur Bildung des zusätzlichen Ausblickfensters in diesem Teil der Wandung. Mit einer solchen Anordnung kann während des Eröffnens der Kapsel durch das zusätzliche Ausblickfenster nicht nur das Implantat selbst, sondern auch die Lage des Implantats zur Verkapselung kontrolliert werden.

Es hat sich als vorteilhaft erwiesen wenn die Ausnehmung in Draufsicht eine etwa ovale Kontur aufweist, da dann einerseits eine genügend große Öffnung realisiert werden und die Verletzungsgefahr gering gehalten werden kann. Zu einer weiteren Verringerung der Verletzungsgefahr dient auch die Abschrägung des stirnseitigen distalen Endes der Schaftwandung in dem dem rinnenförmigen Endteil gegenüberliegenden Bereich.

Um einerseits die Betrachtungsoptik zuverlässig innerhalb des Schaftes festzulegen und andererseits eine Berührung mit dem Behandlungsinstrument zu vermeiden, ist es vorteilhaft, innerhalb des Schaftes ein im Querschnitt etwa D-förmiges Rohr vorzusehen, in das die Betrachtungsoptik eingeführt und in dem diese dann festgelegt werden kann. Für das Behandlungsinstrument kann insbesondere dann, wenn es sich um eine Laserfaser handelt, ein weiteres Rohr zu Führung dieser Faser vorgesehen werden, das innerhalb des Schaftes an der Flachseite des im Querschnitt D-förmigen Rohres anliegt. Wenn dieses Rohr außermittig angeordnet wird, bleibt innerhalb des Schaftes noch Raum zum Einführen eines weiteren Instrumentes. Um eine Kollision zwischen einem solchen weiteren Instrument und der sehr empfindlichen Laserfaser im distalen Bereich zu vermeiden, ist innerhalb des distalen Schaftendes eine Führung für dieses weitere Instrument vorgesehen, und zwar in Form eines Keiles derart, daß dieses weitere Instrument beim Austritt aus dem Schaftende von der Laser-Lichtleitfaser weggerichtet abgelenkt wird.

Innerhalb des Schaftes können, sofern erforderlich, Saug-, Spül- oder andere Kanäle und/oder ggf. auch Rohre vorgesehen sein.

Die Erfindung ist nachfolgend anhand eines in der Zeichnung dargestellten Ausführungsbeispiels näher erläutert. Es zeigen:
- Figur 1: in stark schematisierter Darstellung das erfindungsgemäße Instrument im operativen Einsatz,
- Figur 2: eine Seitenansicht des Instrumentes nach Figur 1 mit im Längsschnitt dargestelltem Schaft,
- Figur 3: einen Schnitt durch den Schaft des Instrumentes nach Figur 2 längs der Schnittlinie III-III,
- Figur 4: in vergrößerter Darstellung den distalen Endbereich des Instrumentenschaftes in Draufsicht,
- Figur 5: einen Schnitt längs der Schnittlinie V-V in Figur 4,
- Figur 6: eine um 90° gedrehte Ansicht des Schaftteiles nach Figur 4 und
- Figur 7: einen Schnitt längs der Schnittlinie VII-VII in Figur 6.

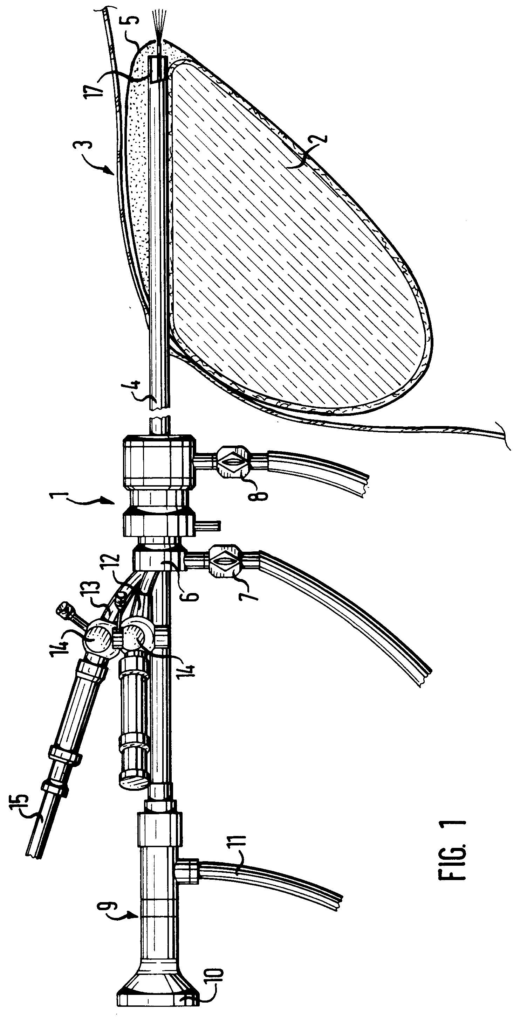

In Figur 1 ist das erfindungsgemäße Instrument 1 so dargestellt, wie es zum Eröffnen eines verkapselten Implantates 2 innerhalb einer weiblichen Brust 3 eingesetzt wird. Die Brust 3 ist im Schnitt dargestellt. Das Instrument ist mit seinem Schaft 4 durch eine Inzision im Bereich der Brustwarze eingeführt. Die zu eröffnende, das Implantat 2 umgebende Kapsel ist mit 5 gekennzeichnet.

Das Instrument 1 als solches entspricht in seinem Grundaufbau einem Hysteroskop. Am proximalseitigen Schaftende 6 sind ein Spülanschluß 7 und ein Sauganschluß 8 vorgesehen, über die Flüssigkeit zum distalen Instrumentenende geführt und auch wieder abgeführt werden kann. Weiterhin ist ein zentraler Kanal zum Einführen und Festlegen einer Betrachtungsoptik 9 vorgesehen, deren Okular 10 sowie Beleuchtungsanschluß 11 in Figur 1 erkennbar ist. Weiterhin sind am proximalseitigen Schaftende 6 zwei Instrumentenkanäle 12 und 13 schräg herausgeführt, die jeweils über einen Hahn 14 absperrbar sind. Innerhalb des Instrumentenkanals 13 ist eine Laserfaser 15 - es handelt sich hierbei um einen Lichtleiter zum Applizieren von Laserlicht - eingeführt, dessen distales Ende am distalen Schaftende liegt und mit dem die eigentliche Durchtrennung erfolgt.

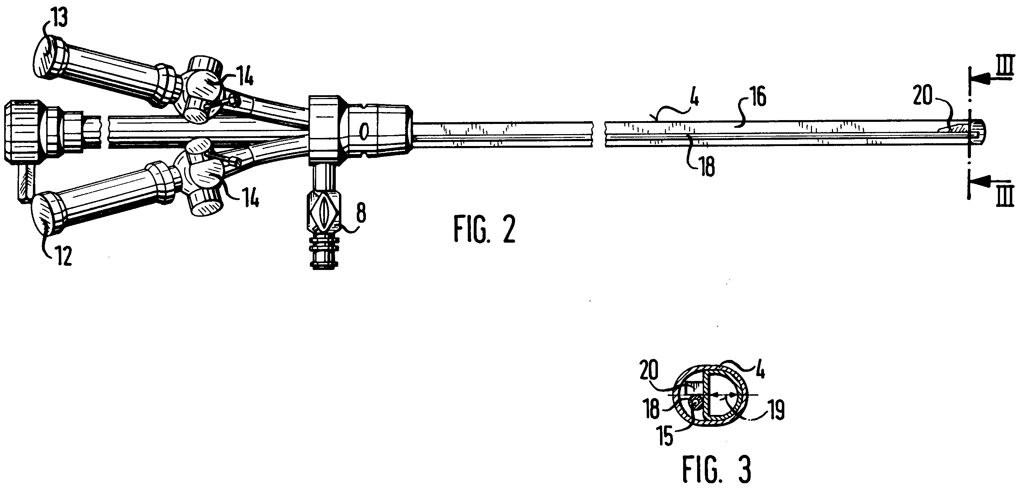

Der Aufbau innerhalb des rohrförmigen Schaftes 4 ergibt sich insbesondere aus der Querschnittsdarstellung in Figur 3. Der Schaft 4 selbst weist einen im wesentlichen ovalen Querschnitt auf, in dem ein im Querschnitt D-förmiges Rohr 16 zur Aufnahme und Festlegung der Betrachtungsoptik 9 angeordnet ist. Das D-förmige Rohr 16 liegt zu der Seite des Schaftes 4, auf der dieser distalseitig zu einem rinnenförmigen Endteil 17 ausläuft und erstreckt sich über mehr als die Hälfte des Schaftquerschnitts, wie aus Figur 3 ersichtlich ist. Der Instrumentenkanal 12 für die Laserfaser 15 ist im Bereich des Schaftes 4 durch ein darin befindliches Rohr 18 gebildet, das kreisförmigen Querschnitt hat und zu einer Seite einer langen Halbachse 19 des ovalen Schaftquerschnitts angeordnet ist, und zwar an der Flachseite des D-förmigen Rohres 16. Das Rohr 18 endet distalseitig im Bereich des rinnenförmigen Endteils 17, kann jedoch auch kurz davor enden, um dem Ende der Laserfaser eine gewisse elastische Verformung zu ermöglichen, um dadurch die Faser vor einem Bruch zu schützen.

Der Instrumentenkanal 12 ist innerhalb des Schaftes 4 nicht gesondert ausgebildet, sondern wird durch den Schaft 4 selbst, dem flachen Teil des D-förmigen Rohres 16 sowie eine Seite des Rohres 18 begrenzt. Nahe dem distalen Schaftende ist innerhalb des Schaftes 4 eine keilförmige Führung 20 angeordnet, die dafür sorgt, daß ein in diesen Kanal 12 eingeführtes Instrument beim Austritt aus dem distalen Schaftende von der Laserfaser 15 abweisend gelenkt wird. Diese Führung 20 ist also vorgesehen, um eine Kollision zwischen Laserfaser und damit eine mögliche Beschädigung der Laserfaser durch dieses Instrument zu vermeiden.

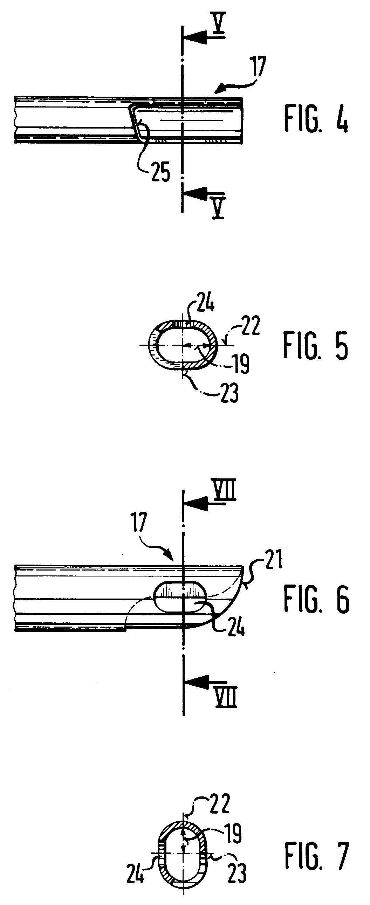

Die Ausbildung des distalen Endes des Schaftes 4 als solches ist den Figuren 4 bis 7 zu entnehmen. Der über den rohrförmigen Vollquerschnitt hinausragende rinnenförmige Endteil 17 ist an seinem distalen vorderen Ende 21 stirnseitig abgeschrägt ausgebildet, wie dies anhand von Figur 6 dargestellt ist, um beispielsweise das Entlangführen des Instrumentes zwischen Kapsel 6 und Implantat 2 zu erleichtern. Wie sich aus den Schnittdarstellungen nach den Figuren 5 und 7 ergibt, ist der rinnenförmige Endteil 17 in seinem Querschnittsprofil bezogen auf die lange Achse 22 des Schaftquerschnittes asymetrisch ausgebildet. Das Rinnenprofil schließt die lange Halbachse 19 ein, reicht zu einer Seite dieser Halbachse 17 bis zur kurzen Achse 23 des Schaftquerschnitts während es zur anderen Seite mit einer Wandung diese deutlich überragt derart, daß sich das Rinnenprofil insgesamt über einen Umfangswinkel von etwa 200° des übrigen Schaftquerschnitts erstreckt. Je nach Anforderungen kann sich das Rinnenprofil auch über einen größeren Umfangswinkel erstrecken.

Dieser rinnenförmige Endteil 17 schützt insbesondere das Implantat vor unbeabsichtigter Kollision mit und somit vor Beschädigung durch das distale Ende der Laserfaser 15 sowie auch durch das distale Ende der Betrachtungsoptik 9.

Dieser Schutz allein reicht jedoch nicht aus, um beispielsweise die das Implantat 2 umgebende Kapsel 5 sicher zu lösen. Es ist deshalb ein zusätzliches Ausblickfenster 24 in dem rinnenförmigen Endteil 17 des Schaftes 4 vorgesehen. Dieses Fenster 24 ist durch eine in Draufsicht etwa ovale Ausnehmung in der Schaftwandung gebildet, wie dies anhand von Figur 6 ersichtlich ist. Die lange Achse dieses Ovals liegt in Achsrichtung des Instrumentes. Das Fenster 24 ist wie aus den Figuren ersichtlich in der die kurze Achse 23 überragenden Flachseite des rinnenförmigen Endteils 17 vorgesehen, es kann jedoch ggf. auch im Boden des Rinnenprofils vorgesehen sein. Die vorstehend beschriebene Ausführungsform hat sich jedoch insbesondere zum Eröffnen der Verkapselung, also zum Durchtrennen der Verkapselung als günstig erwiesen, da mit dem seitlichen Ausblickfenster 24 unter dem sicheren Schutz des Schaftes 4, insbesondere des rinnenförmigen Endteils 17 das Implantat 2 in bezug auf die Kapsel 5 während der Bearbeitung beobachtet werden kann. Die Bearbeitungsstelle an sich wird wie üblich durch die Betrachtungsoptik 9 kontrolliert.

Um das Verletzungsrisiko beim axialen Vorschieben des Schaftes 4 innerhalb des Körpers weiter zu vermindern, ist eine zusätzliche Abschrägung 25 im Bereich des distalen Schaftendes vorgesehen, und zwar an der dem rinnenförmigen Endteil 17 gegenüberliegenden Stirnseite, dort wo der Schaft 4 in den rinnenförmigen Teil 17 übergeht. Diese Abschrägung 25 der Stirnseite ist in Figur 4 dargestellt.

### Bezugszeichenliste

- 1: - Instrument
- 2: - Implantat
- 3: - Brust
- 4: - Schaft
- 5: - Kapsel
- 6: - proximalseitiges Schaftende
- 7: - Spülanschluß
- 8: - Sauganschluß
- 9: - Betrachtungsoptik
- 10: - Okular
- 11: - Beleuchtungsanschluß
- 12: - Instrumentenkanal
- 13: - Instrumentenkanal
- 14: - Hahn
- 15: - Laserfaser
- 16: - D-förmiges Rohr
- 17: - rinnenförmiger Endteil
- 18: - Rohr
- 19: - lange Halbachse
- 20: - keilförmige Führung
- 21: - vorderes Ende von 17
- 22: - lange Achse
- 23: - kurze Achse
- 24: - Fenster
- 25: - Abschrägung

## Patentansprüche

1. Endoskopisches Instrument mit einem rohrförmigen Schaft zum Einführen einer Betrachtungsoptik (9) und eines Behandlungsinstrumentes (15), der in seinem distalen Endteil einen rinnenförmig auslaufenden Teil (17) aufweist, dadurch gekennzeichnet, daß in diesem rinnenförmigen Endteil (17) eine Ausnehmung (24) zur Bildung eines zusätzlichen Ausblickfensters für die Betrachtungsoptik (9) vorgesehen ist.

2. Instrument nach Anspruch 1, dadurch gekennzeichnet, daß der Schaft (4) einen im wesentlichen ovalen Querschnitt aufweist und daß der rinnenförmige Endteil (17) den übrigen Schaftquerschnitt in einem Teil fortsetzt, der eine lange Halbachse (19) des Querschnittovals einschließt.

3. Instrument nach Anspruch 1 oder 2, dadurch gekennzeichnet, daß der rinnenförmige Endteil (17) des Schaftes (4) an seiner distalen Stirnseite (21) eine Abschrägung aufweist.

4. Instrument nach einem der vorhergehenden Ansprüche, dadurch gekennzeichnet, daß sich der rinnenförmige Endteil (17) des Schaftes (4) über einen Umfangswinkel von etwa 200° des übrigen Schaftquerschnitts erstreckt und daß sich die den rinnenförmigen Endteil (17) begrenzende Wandung zu einer Seite der langen Achse (22) des Querschnittovals bis etwa zur kurzen Achse (23) und zur anderen Seite darüberhinaus erstreckt.

5. Instrument nach einem der vorhergehenden Ansprüche, dadurch gekennzeichnet, daß die Ausnehmung (24) in dem Teil der das rinnenförmige Endteil begrenzenden Wandung liegt, der zu einer Seite einer langen Achse (22) des Querschnittovals angeordnet ist, vorzugsweise zu der Seite, auf der sich die Wandung über die kurze Achse (23) des Querschnittovals hinaus erstreckt.

6. Instrument nach einem der vorhergehenden Ansprüche, dadurch gekennzeichnet, daß die Ausnehmung (24) eine etwa ovale Kontur aufweist.

7. Instrument nach einem der vorhergehenden Ansprüche, dadurch gekennzeichnet, daß das stirnseitige distale Ende der Schaftwandung in dem dem rinnenförmigen Endteil gegenüberliegenden Bereich (25) abgeschrägt verläuft, und zwar quer zur Längsachse des Instrumentes.

8. Instrument nach einem der vorhergehenden Ansprüche, dadurch gekennzeichnet, daß innerhalb des Schaftes (4) ein im Querschnitt etwa D-förmiges Rohr (16) zum Einführen der Betrachtungsoptik (9) und als Saug- und/oder Spülkanal angeordnet ist.

9. Instrument nach einem der vorhergehenden Ansprüche, dadurch gekennzeichnet, daß innerhalb des Schaftes (4) ein weiteres Rohr (18) zur Führung einer Laserfaser (15) angeordnet ist, und zwar an der Flachseite des D-förmigen Rohres (16) anliegend.

10. Instrument nach einem der vorhergehenden Ansprüche, dadurch gekennzeichnet, daß innerhalb des distalen Schaftendes jedoch vor dem rinnenförmigen Endbereich (17) innerhalb des Schaftes (4) ein keilförmiges, sich zum proximalen Instrumentenende hin verjüngendes Führungsteil (20) angeordnet ist.
